# EUROPEAN PATENT APPLICATION

(11) **EP 4 016 536 A1**
(43) Date of publication of application: **22.06.2022**
(21) Application number: 20214580.1
(22) Date of filing: 16.12.2020
(51) Int. Cl.: G16H 10/60, G16H 50/30

(54) **BIOMECHANICAL MODELLING OF MOTION MEASUREMENTS**

(71) Applicant: Polar Electro Oy, 90440 Kempele (FI)
(72) Inventor: Korhonen, Topi, 90440 Kempele (FI); Tidemand Larsen, Jacob, 90440 Kempele (FI)
(74) Representative: Kolster Oy Ab

(57) **Abstract**

The present document discloses a solution for modelling biomechanical motion of a human object. According to an aspect, a computer-implemented method comprises: acquiring periodic motion measurement data from a motion sensor located at a determined part of the object while the object is performing periodic motion; acquiring a personal biomechanical model template of the human object, the biomechanical model template defining personal biomechanical characteristics of the object; fitting the motion measurement data with the personal biomechanical model template, the fitting adapting a personal motion style of the object, represented by the motion measurement data, to the personal biomechanical model template, the fitting resulting in an adapted biomechanical motion model describing personalized biomechanical motion of the determined part and at least one other part of the object where no motion sensor is located; determining, on the basis of the adapted biomechanical motion model, at least one parameter describing the biomechanical motion at the other part of the object; and outputting the at least one parameter.

## Description

### TECHNICAL FIELD

The present invention relates to a field of motion sensors arranged to measure biomechanical motion and, in particular, to biomechanical modelling of such motion measurements.

### TECHNICAL BACKGROUND

There are various solutions for measuring motion of a human object, e.g. motion of a hand, body, or foot. The purpose of such measurements may be to measure physical activity or to analyse motion technique, e.g. running technique.

Analysis of musculoskeletal systems of humans or other objects have been investigated in the following publication by Damsgaard M. et al: "Analysis of musculosceletal systems in the AnyBody modeling system", Simulation Modelling Practice and Theory, vol. 14, Issue 8, pages 1100-1111, November 2006. The paper introduces the main features of the system; in particular, the inverse dynamic analysis that resolves the fundamental indeterminacy of the muscle configuration. In addition to the musculoskeletal system, a model can comprise external objects, loads, and motion specifications, thereby providing a complete set of the boundary conditions for a given task.

Another document describing musculoskeletal dynamics is a publication by Ajay Seth et al: "OpenSim: Simulating musculoskeletal dynamics and neuromuscular control to study human and animal movement" published in PLoS Computational Biology in July 2018. It describes construction of a biomechanical model and simulation of motion of the biomechanical model.

### BRIEF DESCRIPTION

The present invention is defined by the subject matter of the independent claims.

Embodiments are defined in the dependent claims.

### BRIEF DESCRIPTION OF THE DRAWINGS

In the following the invention will be described in greater detail by means of embodiments with reference to the accompanying drawings, in which
Figure 1 illustrates a system to which embodiments of the invention may be applied;
Figure 2 an embodiment of a process for estimating user's biomechanical motion;
Figure 3 illustrates a procedure for adapting the process of Figure 2 for various motion styles;
Figure 4 illustrates an embodiment for estimating the biomechanical motion by using a reduced set of motion sensors;
Figure 5 illustrates trajectories of different body parts in connection with measuring user's biomechanical motion;
Figure 6 illustrates a procedure for limiting ranges of measured motion measurement data;
Figures 7A to 7C illustrate embodiments for computing various parameters from an adapted biomechanical motion model derived in the process of Figure 2; and
Figure 8 illustrates a block diagram comprising an apparatus according to an embodiment.

### DETAILED DESCRIPTION OF THE INVENTION

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

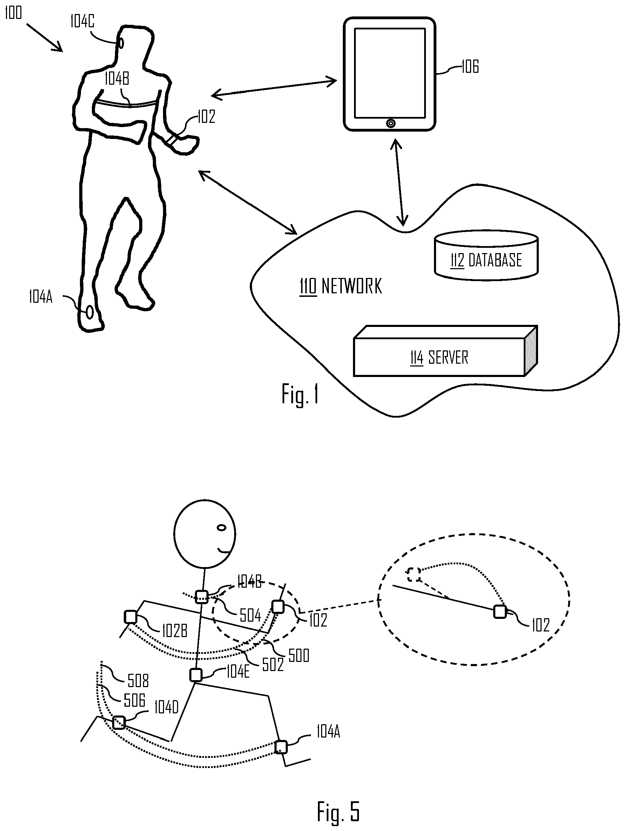

Figure 1 illustrates a system to which embodiments of the invention may be applied. Said system may be used to monitor physical training, activity, and/or inactivity of a user 100. Thus, the embodiments may not be limited to monitoring and/or measuring physical training of the user 100, and thus said system may be used to monitor physical activity and/or inactivity during the day and/or night (e.g. 24 hours a day). Such may be possible using one or more devices described with respect to Figure 1 and in the embodiments below.

Referring to Figure 1, the user 100 may wear a wearable device, such as a wrist device 102, a head sensor unit 104C, a torso sensor 104B, a waist sensor (not shown) and/or a leg sensor 104A. In another example, the wearable device may be and/or be comprised in glasses. In another example, the wearable device is comprised or configured to be coupled with a garment or garments (or apparel). Examples of such garments may include shirt, shorts or trousers, bra(s), swimming apparel, such as swimming suit or cap, and glove(s). The garment or apparel may be worn by the user. In some embodiments, the wearable device is integrated as a part of the garment or apparel.

The wrist device 102 may be, for example, a smart watch, a wearable training computer, sports watch, and/or an activity tracking apparatus (e.g. bracelet, arm band, wrist band). The wrist device 102 may be used to monitor physical activity of the user 100 by using data from internal sensor(s) comprised in the wrist device 102 and/or data from external sensor device(s) 104A-C. The sensor devices may be configured to measure motion of the user 100. The user 100 is an example of a human object subject to the motion measurements in the embodiments described herein. At least some of the motion measurement data may be received from a database 112 via a communication network 110. Such motion measurement data may comprise measurement data measured from the user and/or from other users. The server 114 may be configured to maintain the training database 112 and deliver data between the wrist device and the training database 112. Hence, the database 112 may be used to store motion measurement data of the user 100, for example.

The wrist device 102 may be used to monitor physical activity of the user 100 and/or to be used as a smart watch configured to enable communication with, for example, a portable electronic device 106, the network 110, and/or some other networks, such as a cellular network. Thus, for example, the wrist device 102 may be connected (i.e. wirelessly connected) to the portable electronic device 106, such as a mobile phone, smart phone, tablet and/or computer to name a few. This may enable data transfer between the wrist device 102 and the portable electronic device 106. The data transfer may be based on Bluetooth protocol, for example. Other wireless communication methods, such as Wireless Local Area Network (WLAN) and/or Near Field Communication (NFC), may also be used. The portable electronic device may store and execute an application configured to process the measurement data transferred from the wrist device 102 and/or the server computer 114 to the portable electronic device.

In case of communicating directly with the cellular network, the wrist device 102 may comprise similar communication capabilities as mobile devices, such as 2G, 3G, LTE, LTE-A, 4G and/or 5G communication capabilities. Thus, for example, the wrist device 102 may comprise the communication circuitry capable of operating on said technologies, a Subscriber Identification Module (SIM) and/or a memory comprising a virtual SIM configured to provide a secured identification for the wrist device 102 when operating with the cellular network. It is also pointed out that, in general, the wearable device may comprise a communication circuitry capable of cellular, Bluetooth (e.g. BLE), NFC, WLAN, and/or LAN communication.

The wrist device 102 may be used to monitor physical motion of the user 100. Similarly, the portable electronic device 106 may be used to monitor the motion on the basis of the measurement data received from the wrist device 102 and/or directly from the sensors 104A to 104C.

In an embodiment, the wearable device comprises a motion measurement circuitry configured to measure motion induced by the user 100 to the wearable device. The motion measurement circuitry may comprise one or more motion sensors such as gyroscopes, one or more accelerometers and/or one or more magnetometers. The motion measurement circuitry may use other motion data, such as location data of the user, to determine motion of the user 100. For example, the motion circuitry may comprise a satellite positioning circuitry, such as a global navigation satellite system (GNSS) circuitry. The GNSS circuitry may comprise, for example, a Global Positioning System (GPS) and/or a GLObal NAvigation Satellite System (GLONASS). The satellite positioning circuitry may be used for receiving satellite positioning data. The satellite positioning data may be used, by the wearable device, to determine motion and/or location of the user 100. Other types of motion sensors may be used as well. For example, a system monitoring radio proximity between the devices 102, 104A to 104C may provide means for estimating the motion of the user 100. For example, during running motion sensors attached to both hands or both feet move such that the proximity between the motion sensors changes according to a certain pattern. This pattern may be used as a direct indicator of the motion and motion trajectories of the various body parts of the user where the motion sensors are located.

In an embodiment, any one or more (or all) of the sensor devices 102, 104 to 104C includes at least one of the motion sensor(s) described above: an accelerometer, a magnetometer, a gyroscope, a GPS-based motion sensor, and the radio proximity motion sensor. In an embodiment, the motion sensor comprises multiple motion sensors such as an accelerometer and a gyroscope. The motion sensor may comprise sensor fusion software for combining the measurement data measured by the multiple motion sensors so as to provide physical quantities, such as acceleration data, velocity data, or limb trajectory data in a reference coordinate system having a determined orientation.

The system of Figure 1 may further include a cardiac activity circuitry configured to determine cardiac activity of the user 100, such as heart rate, heart beat interval (HBI) and/or heart rate variability (HRV), for example. The cardiac activity circuitry may comprise an optical cardiac activity sensor unit configured to measure the cardiac activity of the user 100. Example of such sensor may be a PPG (photoplethysmography) sensor. The optical cardiac activity sensor unit may detect the cardiac activity of the user 100 by optical measurement, which may comprise emitting light towards body tissue of the user 100 and measuring the bounced, reflected, scattered and/or emitted light from the body tissue of the user 100. The emitted light may alter when travelling through veins of the user 100 and the alterations may be detected by the optical cardiac activity sensor unit. By using the detected data, the wrist device 102, may determine cardiac activity of the user 100, such as heart rate for example. The optical cardiac activity sensor unit may obtain via the measurement a cardiac activity signal characterizing or carrying the cardiac activity information on the user. As understood, similar cardiac activity circuitry may be comprised in some other wearable device also.

The system may comprise other types of sensor(s). Such sensor(s) may include a Laser Doppler-based blood flow sensor, a magnetic blood flow sensor, an Electromechanical Film (EMFi) pulse sensor, a temperature sensor, a pressure sensor, and/or a polarization blood flow sensor.

As already discussed, the user 100 may use/wear a plurality of motion sensors at various body parts. For example, motion sensors may be provided at one or both hands or multiple sensors per hand (at wrists and arms), one or both feet or multiple sensors per foot, torso, waist and head to measure motion of respective body parts. The motion sensors may transmit data to the same collector device which can be, for example, the wrist device 102 or the PED 106, or potentially both. The collector device may maintain information on the body part to which each motion sensor is attached, as described in some embodiments below.

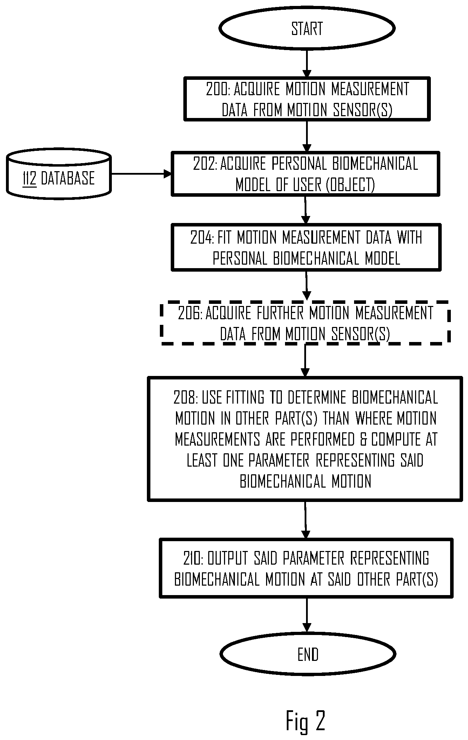

Embodiments described below are related to biomechanical modelling of the user's 100 motion. As described in Background, there exists a biomechanical model to represent biomechanical motion of the user 100 by using the human musculoskeletal characteristics. Embodiments described below utilize such a biomechanical model for the purpose of determining biomechanical motion of the user on the basis of motion measurements performed on the user 100 by using one or more of the motion sensor devices described above. In that manner, the biomechanical model may be adapted to the personal motion style of the user 100, as described below. Figure 2 illustrates a computer-implemented method for modelling the biomechanical motion of a human object, such as the user 100. The method comprises: acquiring (block 200) motion measurement data from a motion sensor located at a determined part of the object; acquiring (block 202) a personal biomechanical model template of the human object, the biomechanical model template defining personal biomechanical characteristics of the object; fitting (block 204) the motion measurement data with the personal biomechanical model template, the fitting adapting a personal motion style of the object, represented by the motion measurement data, to the personal biomechanical model template, the fitting resulting in an adapted biomechanical motion model describing personalized biomechanical motion of the determined part and at least one other part of the object where no motion sensor is located; determining (block 208), on the basis of the adapted biomechanical motion model, at least one parameter describing the biomechanical motion at the other part of the object; and outputting (block 210) the at least one parameter.

In an embodiment, further measurement data may be acquired in block 206, for example when the user is performing an exercise after the fitting, such as a running exercise or an exercise of another motion type. The measurement data acquired in block 200 may also be acquired during one or more training exercises or during a calibration phase for the fitting. In the calibration phase, the apparatus performing the process of Figure 2 may instruct the user to carry out the motion under guidance, e.g. run at a determined speed, walk as regularly as possible, or swim a certain swimming style. The further measurement data may then be acquired under other, uncontrolled conditions. The further measurement data, as mapped to the adapted biomechanical motion model, indicates how the user moved during the exercise without necessarily changing the parameters of the adapted biomechanical motion model, i.e. without recalibrating the adapted biomechanical motion model.

Using the motion measurement data for fitting the personal biomechanical model of the user enables more accurate representation of the user's true biomechanical motion because it is based on both the personal biomechanical characteristics of the user and the measured motion trajectories of the user. After the fitting, the biomechanical model provides the biomechanical inter-relations between different body parts and enables estimation of biomechanical motion of one part based on the motion measurements performed at another body part. Therefore, it is possible to use a reduced number of motion sensors after the fitting.

In an embodiment, the fitting comprises forming an initial motion model from the biomechanical model template, wherein the initial motion model comprises initial motion parameters for a motion type associated with the biomechanical model template, e.g. running, walking, or swimming. From another perspective, the initial motion model may be understood as an example of the biomechanical model template. The initial motion model uses the biomechanical characteristics to define forces and motion trajectories of various body parts according to the motion type. For example, length of the lower limbs and dimensions of them, joints etc. define certain limitations to the possible motion trajectories, and the initial motion model may represent the general motion style with the personal biomechanical characteristics of the user. This initial motion model may then be converted into the adapted biomechanical by using the motion measurement data acquired in block 200. In this manner, the initial motion model is adapted to the user's true motion style, e.g. a running or swimming style or technique.

In an embodiment where the fitting is performed for a body part that is not directly measured with the motion sensor, the fitting may be iterative where the motion trajectory of the body part is gradually adapted so that the initial motion model converges towards the motion measurement data, resulting in the adapted biomechanical motion model. For example, if the hip motion is inferred from the motion measurement data measured from the foot/feet, the adaptation may comprise analysis of the foot motion measurement data and inferring hip motion on the basis of the initial motion model and the foot motion readily adapted to the foot motion measurement data. Subsequently, new parameters describing the hip motion may be computed. However, the changed parameters describing the hip motion may cause a change to parameters that describe musculoskeletal motion of thighs and subsequently the feet. By analysing this change and comparing the result with the foot motion measurement data, a new set describing the hip motion may be computed that results in a better match with the motion measurement data. In this manner, the fitting may iteratively discover parameters for all the body parts such that the match with the motion measurement data is acquired and the adapted biomechanical motion model is produced.

In an embodiment, for the purpose of the fitting the initial motion model and the measurement data may be mapped to a common coordinate system. For example, the motion measurement data may represent the motion in an x,y,z coordinate system of the motion sensor that is bound to a reference point, e.g. the hand or foot and not to a global coordinate system. Accordingly, the coordinate system may move along the movement of the reference point. The initial motion model may be mapped to the same coordinate system to enable the fitting.

In an embodiment, the motion measurement data is filtered before the fitting. For example, averaging may be performed to remove natural inconsistencies in the motion. Since the motion is periodic, the motion measurement data may be divided into periodic sets, and the averaging may be performed across the periodic sets to derive an average motion trajectory within a period for the fitting.

In an embodiment, the initial motion model may define a simulation model comprising parameters for simulating or visualizing the motion style that is based on only the biomechanical model template of the motion style, i.e. before adapted by using the motion measurement data. The parameters for the simulation model may then be adapted in block 204 to the user's true motion style.

The motion measurements may be divided into two logically separate measurement phases where different sets of sensors are used: measuring the motion for the fitting performed in block 204 and measuring the motion in block for the purpose of estimating the biomechanical motion in a particular exercise the user has conducted without iterating block 204. For the purpose of the fitting, the user may wear multiple motion sensors (e.g. two to eight) in pre-defined body parts while performing a certain type of motion, e.g. running or walking. As described above, the initial motion model based on the biomechanical model template may comprise parameters that define the user's motion under the condition of the user's personal characteristics used as inputs to the biomechanical model template. As known, users with very similar personal characteristics may adopt very different motion styles, e.g. running style or walking style. Therefore, the motion measurement data acquired in block 200 may be used to match the initial motion model with the true motion of the user. Therefore, the parameters initial motion model, based on the biomechanical model template acquired in block 202, are modified so that the motion simulated with the adapted biomechanical motion model (resulting from the fitting) matches with the measured motion. After the fitting, the initial motion model is modified towards the true motion of the user, thus improving the accuracy of modelling the user's real motion.

After the fitting has been performed, a reduced set of motion sensors may be used. As described in some embodiments below, it may be sufficient to have a single motion sensor in either hand/arm or foot to acquire representation for the biomechanical motion of both hands/arms or feet, respectively. Depending on the motion type, a certain (reduced) set of motion sensors may be needed to get full representation of the biomechanical motion of the whole body. For example, to get the full representation of the biomechanical motion of the whole body for running, walking or swimming after the fitting, it may be sufficient to have the motion sensor in either hand/arm and in either foot. For swimming, a head motion sensor may be used to acquire the further measurement data. The minimum number of motion sensors required may be determined separately for each type of motion and indicated to the user at a beginning of the motion measurements. Higher accuracy can naturally be achieved with a higher number of motion sensors. For example, in swimming the motion sensor may be provided in both hands but only in one of the feet after the fitting, and the fitted biomechanical model enables full representation of both feet after the fitting. In connection with some motion types, only a single motion sensor attached to the body part having the greatest degree of motion may be sufficient to get the full representation of the biomechanical motion of the whole body. The adapted biomechanical motion model defining the user's personal motion style may be used to compute the motion trajectories for all the other body parts on the basis of a single measurement point or a reduced set of measurement points.

In an embodiment, the fitting has a capability of adapting to the number of motion sensors providing the measurement data for the fitting. For example, for optimal fitting for running, the motion sensors could be provided in all limbs, e.g. two motion sensors per limb (one at the end and one in a middle part of the limb), and at least one motion sensor attached to the torso (waist and/or chest). Since the biomechanical model template defines the musculoskeletal characteristics of the user, a reduced set of motion sensors may still provide an acceptable result for the fitting. Basically, any number of motion sensors may be suitable for the fitting at least on some degree. For example, if there is a motion sensor only in the hand and in the torso, accurate adaptation may be acquired for the hand(s) and the torso. Additionally, improvement to the initial motion model may be acquired for the other body parts as well, thanks to the inter-dependencies of the body parts defined by the biomechanical model template. In an embodiment, the personal biomechanical characteristics comprise at least a mass of the user, body composition of the user, body dimensions of the user, and a fitness metric of the user. Further personal biomechanical characteristics may include muscular elasticity and force generation capability, and joint mobility. These parameters may be measured from the user and stored in the biomechanical model template. For example, the muscular characteristics may be measured by using one or more electromyography sensors, and the muscular elasticity and the joint mobility may be measured manually or by using appropriate sensors. The user may input to his/her user account stored in the database 112 the above-described personal characteristics. Some of the personal characteristics may be measured and acquired in connection with block 200. The body composition may represent the amount of liquid, fat, muscle etc. in the body. The body dimensions may include lengths of the user's limbs such as an arm length, a leg length, circumferences of the limbs, dimensions of the torso, total height etc. The fitness metric may represent the fitness. The fitness metric may affect the motion trajectories during running, e.g. contact time to the ground, limb trajectories, limb velocities etc. The personal biomechanical characteristics may be used to build the initial motion model for each type of motion, e.g. according to the musculoskeletal model mentioned in Background. The musculoskeletal model may additionally model the joints and their physical characteristics such as elasticity of the joints and personal muscular properties, as described above. As described above, the initial motion model is based on only the user's personal biomechanical characteristics and not yet mapped to the true motion trajectories or motion style of the user, and this is provided by the fitting in block 204.

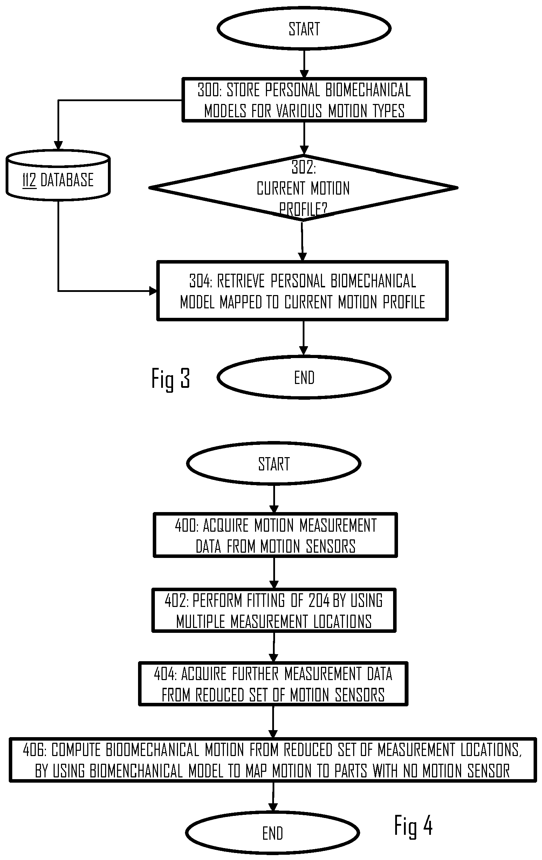

In an embodiment, a plurality of personal biomechanical model templates or initial motion models is stored in the database 112, each mapped to a different motion profile representing a certain type of motion. A current motion profile of motion the user may then be determined and the personal biomechanical model or initial motion model mapped to the current motion profile retrieved from the database 112 in block 202. Figure 3 illustrates such an embodiment. Referring to Figure 3, the biomechanical model templates may be stored (block 300) in the database 112 (or in another memory) for various motion types including, for example, running, walking, swimming, skiing, cycling, paddling, and fitness training. The motion types may be periodic motion types where the same motion trajectory is repeated over and over. With respect to the fitness training, the motion type may be fitness or strength training where the same motion is repeated, e.g. a handle ball training, sit-up training, push-up training, cheer dip training, and squat training. Each personal biomechanical model template may be stored with an indicator indicating the motion type of the template. The template may readily include the personal characteristics of the user 100 and define default motion trajectories for a person with such personal characteristics, as described above. In block 302, the apparatus performing the fitting in block 204 determines the motion type (motion profile) of the motion represented by the motion measurement data acquired in block 200. The determination may be based on user input indicating the motion type (e.g. running), or it may be determined from motion analysis applied to the motion measurement data. As known in the art, the different motion types induce different types of motion measurement data with characteristics unique to each motion type, and a motion detection algorithm may be applied to the motion measurement data to detect the motion type. Yet another embodiment for determining the motion type is the user's current location or training schedule. For example, if the user's training schedule indicates a running exercise or that the user has started a running exercise in his/her training computer (the wrist device 102), it may be determined that the motion is running (similar to other motion types). If the location as determined from satellite navigation system coordinates indicates swimming hall, the motion type may be determined as swimming (respectively to other motion types and associated locations). Upon detecting the motion type, the apparatus may retrieve the corresponding biomechanical model template from the database 112 (or memory) in block 304. Thereafter, the fitting of block 204 may be performed for the retrieved biomechanical model template.

In an embodiment, the motion measurement data for the fitting is acquired from multiple motion sensors located at different parts of the object, as described above, and the fitting is performed by using the motion measurement data acquired from the multiple motion sensors. Thereafter, a reduced set of motion sensors may be used to determine the motion at even those body parts that are not directly measured after the fitting. Figure 4 illustrates a procedure according to such an embodiment. Referring to Figure 4, the motion measurement data from the multiple motion sensors attached to the different body parts of the user 100 are acquired in block 400. Block 400 is an embodiment of block 200. In block 402, the fitting of block 204 is performed by using the motion measurement data acquired from the multiple measurement locations. The higher the number of measurement locations, the more accurate fitting with the true biomechanical motion is achieved for the adapted biomechanical motion model personalized for the user 100. For example, if the motion measurement data in block 200 is acquired only from hand measurements, the fitting in block 204 may be performed only for the hand motion and, optionally, for the torso motion because the torso motion may at least to some degree inferred from the hand motion when the user's personal characteristics have been stored in the biomechanical model template. However, the feet motion may be taken directly from the biomechanical model template or initial motion model without any fitting with measurement data. When the feet motion is also measured in block 400, the fitting in block 402 may be performed also for the feet motion, thus providing more accurate biomechanical motion model after the fitting.

After the fitting, it may be sufficient to acquire the motion measurement data from a reduced set of sensors to determine the motion at even those body parts that are not directly measured. In block 404, further measurement data is acquired from the reduced set of motion sensors. For example, if the motion sensor was attached to both hands or both feet in block 400, the reduced set of motion sensors may include a motion sensor in only one foot and/or in only one hand. As described above, the biomechanical motion model defines the (skeletal or musculoskeletal) inter-relations between the body parts and thus provides the means for inferring the motion of one body part on the basis of measurement data acquired from another body part. In block 406, the biomechanical motion is computed from the further motion measurement data. The further motion measurement data may be used in combination with the adapted biomechanical motion model as follows. For the body part(s) associated with the further motion measurement data, the biomechanical motion model for that/those body part(s) may be taken directly from the further motion measurement data to represent the true motion of that/those body part(s). However, the fitted biomechanical motion model may be used to map the further measurement data to the biomechanical motion of the other body part(s) for which there is no further measurement data. Accordingly, the motion to such body part(s) may not be taken directly from the adapted biomechanical motion model but as biased on the basis of the further measurement data. For example, if the adapted biomechanical motion model for running defines direct forward-backward hand motion trajectories but the further motion measurement data measured from left hand indicates slight left-right motion as well, the biasing may include adding the same left-right motion to the right hand motion, with an opposite phase of course. If the personal biomechanical model template defines left-right asymmetry in the personal characteristics of the user 100, e.g. one arm is shorter than the other, the asymmetry may be brought into the bias by the biomechanical motion model. Because of the asymmetry, the trajectories of the hands or feet may differ because of different physical proportions, and this may be applied to both the fitting and block 406.

In an embodiment, the at least one parameter determined in block 208 and output in block 210 comprises animation of the user's 100 motion as determined on the basis of the adapted biomechanical motion model. The animation may include animation of the motion of the part of the object that is directly measured by a motion sensor and for which the measurement data is available. Additionally, the animation may include animation of the motion of the other part(s) of the object for which there is no directly measured motion measurement data available. As described above, the biomechanical motion for the other body part(s) may be determined by using the biomechanical motion model. Figure 5 illustrates trajectories of the different body parts, namely the trajectories 500, 502 for the hands, trajectories 506, 508 for the feet, and a trajectory 504 for a torso. At least some of the trajectories may be measured directly by the motion sensors described above. Figure 5 illustrates further motion sensors attached to both feet (sensor 104D) and to a waist (sensor 104E). The exact number and locations of the motion sensors may vary according to the motion style, as described above. The fitting may also be adaptive, as described above, and perform the fitting when a reduced set of motion sensors is available. If it is determined in the fitting that the user's motion is symmetric for both hands and feet, the trajectories 500 and 502 and trajectories 506 and 508 may also be symmetric and have substantially the same trajectories, juts with opposite phases. Because of the repeated motion styles, the motion of the torso may also follow the motion of the hands and/or feet. Therefore, the motion of the torso may also be animated when the sensor 104B is not attached to the user while acquiring the further measurement data in block 206. The biomechanical motion model may define the inter-relation between the motion trajectory of the torso and the motion trajectories of the hands and/or feet.

In an embodiment, the biomechanical model template and/or the adapted biomechanical motion model is used to limit a range of possible values of the (further) motion measurement data. As known from the human anatomy, certain body parts are capable of moving along certain limited trajectories. For example, the elbow does not allow the arm to turn over about 180 degrees. Therefore, the physical limitations may be used to limit the range of possible values for the motion sensor 102 attached to the arm or wrist or hand of the user (as illustrated in the dash-circled picture in Figure 5. Similarly, the physical characteristics of the other joints or body parts may be used to limit the possible motion values of the measurement data of the other motion sensors. For example, only a certain degree of lateral (left-right) motion may be allowed for motion measurement data acquired from the motion sensor(s) attached to the user's foot in connection with running motion type. The obvious reason is the that conventional running (with periodic stepping) is primarily directed to forward-backward motion of the feet.

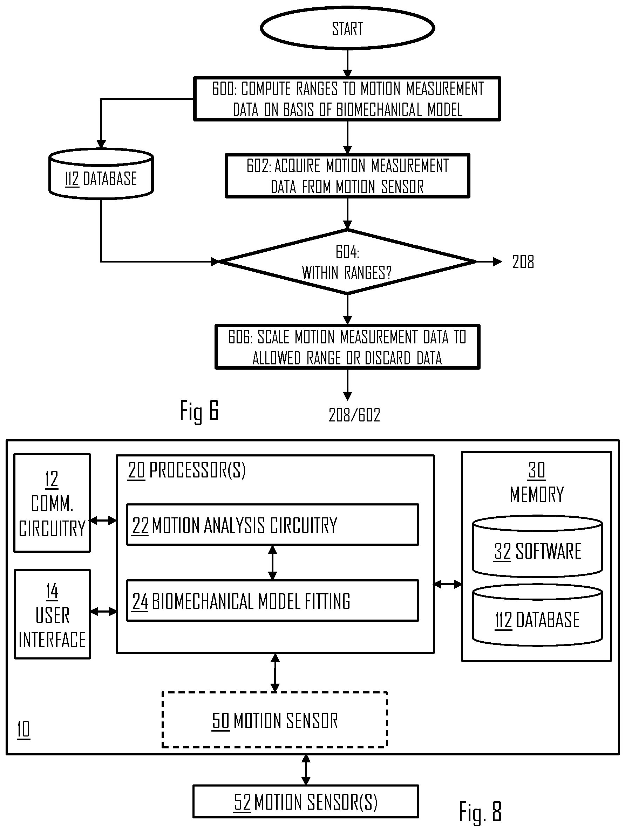

Figure 6 illustrates a process for limiting the ranges and using the limited ranges for the (further) motion measurement data. Referring to Figure 6, the allowed ranges for the motion measurement data associated with at least one body part is computed in block 600 on the basis of the personal biomechanical model template or the initial motion model. The allowed ranges may be determined on the physical limitations to the motion of the different body parts, as described above. Different body parts may be associated with different limitations to the ranges of the motion measurement data. The limited ranges may be stored in the database or in the memory, or configured to the biomechanical motion model template. In block 602, motion measurement data is acquired from a motion sensor attached to a determined body part. The motion measurement data is then cross-referenced with the allowed ranges for the particular body part in block 604. Block 604 may include retrieving the allowed ranges for the particular body part from the database 112 or from the memory for the comparison. If the measurement data is determined to be within the ranges, the measurement data may be forwarded to block 208. However, if the measurement data is found to fall outside the allowed range, the process proceeds to block 606 wherein the measurement data falling outside the allowed ranges is either mapped the allowed range or the measurement data is discarded. The mapping may include mapping only the measurement data values falling outside the allowed range so that they become mapped within the allowed range, or mapping also other values so that the motion measurement data is unanimous in the sense that the mapping does not cause unnatural representation of the motion. Thereafter, the procedure may continue in block 208 in case there is still measurement data to be used in block 208. If all the measurement data is discarded in block 606, the process may return to block 602 to acquire more motion measurement data.

In an embodiment, the ranges for the motion measurement data are limited by using a centre of gravity of the user 100 as a reference. For example, the allowed ranges for the motion measurement data may be bound to the centre of gravity such that the motion cannot reach such values that would indicate that the user would fall or lose balance, because that would not represent the true intended motion. Such a limitation would exclude any measurement data measured under a condition where the user has stumbled or fallen. Therefore, such measurement data would not corrupt the biomechanical motion model.

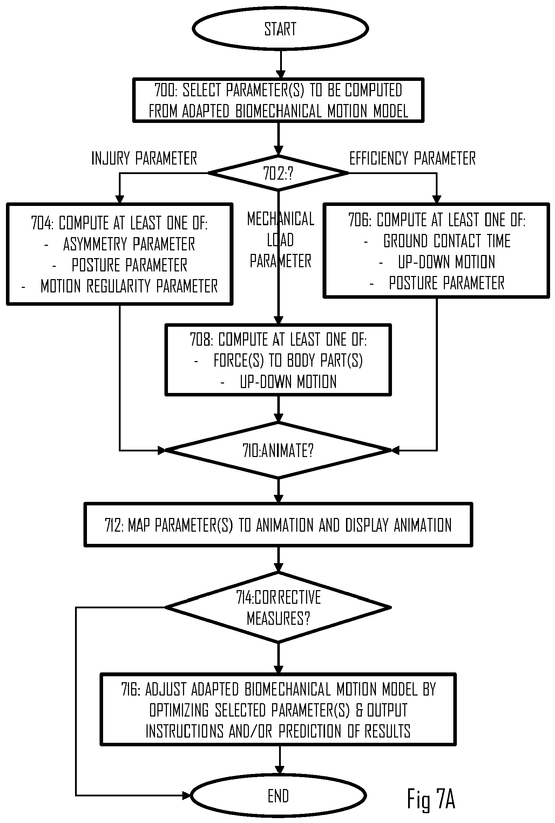

Let us then describe some embodiments of the parameters that may be computed based on the adapted biomechanical motion model. Figure 7A illustrates an embodiment of a procedure for computing the various parameters and implementing the parameters to the animation that illustrates the effect of the various parameters. Figure 7A illustrates an embodiment of block 208 that can be executed directly in connection with blocks 200 to 204 or thereafter in a separate procedure that may be performed by the same apparatus that performed blocks 200 to 204 or another apparatus.

Referring to Figure 7A, after acquiring the adapted biomechanical motion model, the at least one parameter described below is selected for computation in block 700. The selection may apply to multiple parameters and multiple of the three branches illustrated in Figure 7A. The parameters to be computed may be categorized into categories, e.g. injury parameters, mechanical load parameters, and motion efficiency parameters. Some of the computed parameters may belong to multiple categories, as illustrated in Figure 7A.

In an embodiment of block 704 illustrating injury parameters that may be computed on the basis of the adapted biomechanical motion model, the parameters include at least one of the following: a motion asymmetry parameter, a posture parameter, and a motion regularity parameter. The asymmetry parameter may indicate left-right asymmetry in the motion of the user 100. This parameter may be determined in connection with performing the fitting, e.g. from the adapted biomechanical motion model. The asymmetry parameter may be computed, for example, by comparing the motion of a left limb with the motion of a right limb. If there is a difference higher than a threshold, the asymmetry may be detected, and the parameter may describe a degree of the asymmetry. The asymmetry may be determined from the deviation between the motion trajectories of the motion measurement data acquired from the motion sensors attached to the different hands and/or feet. The motion measurement data may be bound to the same coordinates and, as a consequence, the asymmetry can be detected from the motion measurement data. For example, a lateral component in the (running) motion measurement data acquired from one hand/foot may be larger than in the motion measurement data acquired from the other hand/foot.

The posture parameter may be computed by analysing the user's posture during the motion. The parameter may describe, for example, whether the user is leaning back, forth, or to a side during the motion and, additionally, a degree of such leaning.

The motion regularity parameter may be computed to determine whether or not the periodic motion represented by the measurement data is regular. This may be computed by estimating a deviation or variance in the periodicity, for example. The motion regularity parameter may indicate the degree of (ir) regularity in the motion.

In an embodiment of block 706 illustrating motion efficiency parameters that may be computed on the basis of the adapted biomechanical motion model, the parameters include at least one of the following: a ground contact time, cadence, a degree of up-down motion, and the posture parameter described above in connection with block 704. The ground contact time may be computed from the motion measurement data used for fitting and/or from the further measurement data. The contact time for one foot may be computed from the measurement data acquired for the other foot, taking into account the potential left-right asymmetry between the feet proportions and the stepping, as defined by the adapted biomechanical motion model. After the fitting, the contact time may be computed equally from the motion measurement data acquired from the torso sensor or even from the hand sensor(s). In the biomechanical motion model, the contact start time and contact stop time of a foot may be mapped to a certain position or motion of the hand and, upon detecting the respective position or motion of the hand in the hand motion measurement data, the contact time(s) of the foot/feet can be computed.

The cadence is an indicator of running efficiency. The cadence may be computed directly from the periodicity of the motion.

The degree of up-down motion is a direct indicator of running efficiency. As a consequence, the degree of overall up-down motion in the adapted biomechanical motion model may be directly mapped to the running efficiency. The up-down motion may be computed from the motion of any body part, but the most accurate estimate may be acquired from the torso or waist motion measurement data. As another example, the adapted biomechanical motion model may directly indicate a step size, and the step size may be compared with a reference step size or a reference step size range deemed optimal for the person with the biomechanical characteristics (e.g. length of lower limbs) of the user to derive the efficiency. As described above, the posture parameter indicates whether or not the user is leaning overly forward, side, or backward during the motion. Such a factor may serve as an indicator of inefficiency as well as an injury indicator. In a similar manner, a running power may be computed on the basis of the biomechanical motion model and, optionally, the further motion measurement data.

In an embodiment of block 708 illustrating mechanical load parameters that may be computed on the basis of the adapted biomechanical motion model, the parameters include at least one of the following: the degree of up-down motion and an impact force on one or more body parts, e.g. a joint or a ligament. The parameter describing the impact force may indicate an impact of the motion on a joint (or ligament) of the user 100, e.g. a degree of said impact. The adapted biomechanical motion model may describe the rigidness or elasticity of the various body parts including the muscles and joints, optionally personalized to the user's personal biomechanical characteristics such as the muscular elasticity, and the motion measurement data together with the adapted biomechanical motion model may indicate an impact force when running, for example. Via the adapted biomechanical motion model, an impact force computed from the motion measurement data acquired from the foot, torso, or even the hand may be mapped to an impact force on a joint, e.g. a knee or ankle via the adapted biomechanical motion model. The parameter output may thus be proportional to the impact force on the joint and/or other body parts.

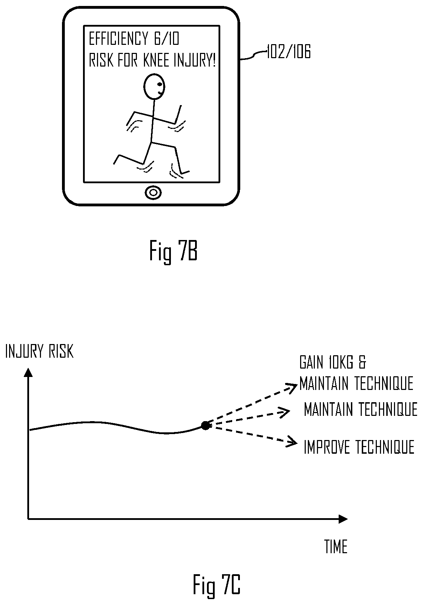

In an embodiment, the one or more parameters computed in blocks 704 to 708 may be used for animating the user's motion. In block 710, it is determined whether or not to play an animation of the user's motion represented by the motion measurement data. If the animation is determined to the played, the computed parameter(s) may be used as inputs to the animation generation. For example, the asymmetry parameter describing the degree of asymmetry may be used to highlight the asymmetry in the animation. The animation of the body motion as such may follow the adapted biomechanical motion model while the parameters may determine what type of side information is output during the animation. For example, if the asymmetry parameter indicates the presence of asymmetry, a textual output indicating the asymmetry may be provided. Similarly, the ground contact time may be output as well as a metric describing the efficiency in general on a scale. The animation may be displayed on a display unit of the wrist device 102 or the portable electronic device 106 such as a smart phone or a (tablet) computer, as illustrated in Figure 7B.

In an embodiment, block 704 further comprises computing, by using the parameters computed in block 704, a metric describing a risk for injury for the user. The metric may be computed by using the parameters computed in block 704 and, additionally, parameters computed in block 706 and/or 708. For example, the mechanical load may have a great effect on the risk for injuries, as well as the degree of up-down motion. The metric may be compared with a threshold. If the metric indicates a risk factor greater than the threshold, the apparatus may output a warning indicating an increased risk for injuries in connection with the animation or in another manner. Similarly, an efficiency metric may be computed in block 706 on the basis of the respective parameters. The metric(s) may be output in connection with the animation. The risk for injuries may be analysed from the adapted biomechanical motion model, and parameter describing the risk may include a metric indicating a risk factor indicating the risk and, optionally, what type of injury is potential for the user. For example, if the adapted biomechanical motion model indicates that the user lands heavily on his/her heel when running, the parameter may indicate a high risk for heel or knee injury. As another example, if the up-down motion of the user is higher than a threshold, the parameter may indicate a risk for injury. The impact force may be used as one parameter for the risk evaluation.

From another perspective, the parameters of the adapted biomechanical motion model describing the user's motion style may be analysed in terms of reference motion style(s) associated with injuries, e.g. on a basis of a cohort study. If the parameters indicate that the motion style has correlation with a motion style associated with injuries, the output parameter may indicate the risk for injury. As described herein, the apparatus may output an animation or another instruction as how to change the motion style to reduce the risk for injuries.

The apparatus performing the process of Figure 7A may also output at least one instruction to the user as how to correct the motion in order to reduce the risk for injuries, to improve the efficiency, and/or to reduce the mechanical load on the body parts. In another embodiment, the apparatus may use the parameters computed in blocks 704 to 708 to estimate future development. Block 714 comprises determining whether or not to determine the corrective instructions and/or to perform the prediction. Upon determining to do so, block 716 may be executed. For example, the at least one instruction may include a corrective adjustment to the adapted biomechanical motion model to remove the asymmetry and display the animation with the corrected asymmetry so that the user gets a visual idea as how to correct the asymmetry. The animation may also alternately display the motion with the asymmetry and with the corrected/removed asymmetry to improve the illustration. Similarly, the apparatus may output an animation or another instruction as how to change the motion style to reduce the risk for injuries. The animation may be derived by adapting the adapted mechanical motion model (iteratively) towards a direction where a force acting on a joint or a body part reduces, thus reducing the risk for the injury. The adaptation may be made under the conditions that the speed, efficiency, or other factors of the motion would still remain and that the forces acting on other body parts would not substantially increase. Similarly, the apparatus may output an animation or another instruction indicating how to change the motion style to improve the efficiency, e.g. to change the posture towards a determined direction, to change the cadence, etc.

With respect to the prediction of the future development, block 716 may comprise predicting, on the basis of the various parameters described herein, a future value or values of the respective parameters, under various scenarios. For example, the risk for injuries may be projected into the future by using the current motion style, as indicated by the adapted biomechanical motion model and the respective parameters. Additionally, the apparatus may change the value of at least some of the parameters and/or at least some of the user's personal characteristics to realize alternative scenarios. Figure 7C illustrates such a projection for the risk for injuries under various scenarios.

Referring to Figure 7C, the apparatus may monitor the risk for injuries over a certain time interval for which the motion measurement data is available. The time interval may span over multiple days or weeks and over multiple exercises performed by the user. Accordingly, the projection of the risk for injuries in a scenario where all the parameters remain (the user maintains the motion style and other factors) is a straightforward extrapolation. In order to create an alternative scenario, the apparatus may change at least one parameter and recompute the effect on the risk for injuries and make the projection with the changed parameter(s) and respective effect. For example, the apparatus may change the user's weight and recompute the adaptive biomechanical motion model and/or the parameters. For example, the weight affects directly the forces acting on the joints or how much weight for the up-down motion is given in the risk for injuries computation. Reduction in the weight lowers the risk for injuries and, thus, causes projection indicating a lower risk for injuries in the future. On the contrary, increased weight may raise the projected risk for injuries, as illustrated in Figure 7C, provided that the motion style (technique) remains the same. As another example, the apparatus may use the teachings described herein to artificially change the adapted biomechanical motion model and to recompute the risk for injuries. For example, the apparatus may determine what type of injury is the most potential for the user by using the mechanical load parameters, for example. Upon finding a body part that is prone to injury with the current motion style, the apparatus may start readapting the biomechanical motion model towards a direction where the mechanical load on the body part reduces (without significantly increasing mechanical load in other body parts). Upon finding an improved motion style, the apparatus may recompute the parameter(s) and project the risk for injuries into the future with the recomputed parameter(s), as illustrated in Figure 7C.

A similar procedure may be performed for the efficiency, for example. The apparatus may adapt the biomechanical motion model such that the inefficiencies in the posture, cadence, etc. are reduced and recompute the parameters and project the resulting efficiency into the future in order to estimate an effect of the changed motion style.

In an embodiment, the fitting calibrates the personal biomechanical model with the motion measurement data, thus resulting in the biomechanical motion model personalized to the user's 100 motion style. The fitting may be repeated to recalibrate the personal biomechanical (motion) model to potential changes in the motion of the object. For example, the user's running style may improve as the user's fitness improves, motion style changes, or the user's personal characteristics such as the weight change. The earlier biomechanical motion model(s) may be stored in the database so that the user can observe how the motion has changed over time. The calibration may be performed by repeating blocks 200 to 204 of Figure 2 by using new motion measurement data. The apparatus may also perform a predictive animation of the user's motion with the condition that one or more of the personal characteristics change. For example, the apparatus may perform the refitting by changing the user's weight and/or body composition and performing the refitting with the same motion measurement data that has been readily acquired in block 200. Because the user's biomechanical model template changes, so does the biomechanical motion model after the fitting. This gives the user an idea as what could be the effect of the changed personal characteristics on the motion.

Figure 8 illustrates an embodiment of an apparatus configured to perform the process of Figure 2 or any one of the embodiments thereof. In an embodiment, the process of Figure 2 is performed by a processing system comprising at least one processor and at least one memory comprising a computer program code readable by the at least one processor. The computer program code may form a computer program product defining a computer process executed by the at least one processor, when the at least one processor reads and executes the computer program code. Referring to Figure 1, the process of Figure 2 may be carried out by the wrist computer 102, the portable device 106, the server computer 114, or by a combination of two or more of these devices.

Referring to Figure 8, the apparatus may comprise the at least one processor 20 and the at least one memory 30. The computer program code 32 may be stored in the memory. The memory 30 may further store the database 112, or the database may be stored elsewhere by be accessible to the apparatus, e.g. via the network 110. The apparatus may further comprise a communication circuitry 12 supporting at least one communication protocol. The communication circuitry 12 may support multiple protocols, e.g. Bluetooth for communication with the motion sensors described above, Universal Serial Bus (USB) communications, IEEE 802.11 standard (WiFi), a cellular communication protocol or protocols, and/or other wired and/or wireless protocols. The apparatus may further comprise a user interface comprising a display unit, a loudspeaker, one or more user input devices, etc. In embodiments where the apparatus is the wrist computer, the apparatus may comprise a motion sensor 50 configured to measure motion measurement data from the user's wrist or arm or hand. In embodiments where the apparatus is the portable device 106 or the server 114, the apparatus may employ only external motion sensors 52. The external motion sensors may include at least some of the motion sensors 104A to 104C, 102B described above.

The at least one processor 20 may include a biomechanical model fitting circuitry 24 configured to perform at least blocks 200 to 204 of Figure 2. The circuitry 24 may thus carry out the calibration of the personal biomechanical motion model with the motion measurement data to acquire the biomechanical motion model adapted to the user's true motion. The processor(s) 20 may further include a motion analysis circuitry 22 configured to use the fitted biomechanical motion model to analyse the (further) motion measurement data received from the motion sensor(s) 50, 52 according to any one of the above-described embodiments.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), graphics processing units (GPUs), processors, controllers, micro-controllers, microprocessors, other electronic units designed to perform the functions described herein, or a combination thereof. For firmware or software, the implementation can be carried out through modules of at least one chipset (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, etc., described with regard thereto, and they are not limited to the precise configurations set forth in the given figures, as will be appreciated by one skilled in the art.

As described above, the processes or methods described in Figures 2 to 7 or any of the embodiments thereof may also be carried out in the form of one or more computer processes defined by one or more computer programs. A separate computer program may be provided in one or more apparatuses that execute functions of the processes described in connection with the Figures. The computer program(s) may be in source code form, object code form, or in some intermediate form, and it may be stored in some sort of carrier, which may be any entity or device capable of carrying the program. Such carriers include transitory and/or non-transitory computer media, e.g. a record medium, computer memory, read-only memory, electrical carrier signal, telecommunications signal, and software distribution package. Depending on the processing power needed, the computer program may be executed in a single electronic digital processing unit or it may be distributed amongst a number of processing units.

It will be obvious to a person skilled in the art that, as the technology advances, the inventive concept can be implemented in various ways. The invention and its embodiments are not limited to the examples described above but may vary within the scope of the claims.

## Claims

1. A computer-implemented method for modelling biomechanical motion of a human object, comprising:
acquiring periodic motion measurement data from a motion sensor located at a determined part of the object while the object is performing periodic motion;
acquiring a personal biomechanical model template of the human object, the biomechanical model template defining personal biomechanical characteristics of the object;
fitting the motion measurement data with the personal biomechanical model template, the fitting adapting a personal motion style of the object, represented by the motion measurement data, to the personal biomechanical model template, the fitting resulting in an adapted biomechanical motion model describing personalized biomechanical motion of the determined part and at least one other part of the object where no motion sensor is located;
determining, on the basis of the adapted biomechanical motion model, at least one parameter describing the biomechanical motion at the other part of the object; and
outputting the at least one parameter.

2. The computer-implemented method of claim 1, further comprising:
storing a plurality of personal biomechanical model templates, each mapped to a different motion profile representing a certain type of motion;
determining a current motion profile of motion of the object and retrieving the personal biomechanical model mapped to the current motion profile.

3. The computer-implemented method of claim 1 or 2, wherein the determined part of the object is one hand or one foot of the object and the other part is the other hand or the other foot of the object, respectively.

4. The computer-implemented method of claim 3, wherein the at least one parameter describes left-right asymmetry in the motion of the object.

5. The computer-implemented method of any preceding claim, wherein the at least one parameter comprises animation of the motion of the determined part of the object and animation of the motion of the other part of the object.

6. The computer-implemented method of any preceding claim, further comprising:
limiting, on the basis of the fitting, a range of possible values of the motion measurement data or further motion measurement data acquired after the fitting; and
upon detecting a value of the motion measurement data or further motion measurement data falling outside the range, mapping the value to the range or discarding the value.

7. The computer-implemented method of any preceding claim, wherein said determining the at least one parameter comprises determining an impact of the motion on a joint of the object, and wherein the at least parameter indicates a degree of said impact.

8. The computer-implemented method of any preceding claim, further comprising:
acquiring motion measurement data from multiple motion sensors, including said motion sensor, located at different parts of the object;
performing the fitting by using the motion measurement data acquired from the multiple motion sensors;
determining, on the basis of the fitting, the biomechanical motion at the other part of the object by using further motion measurement data acquired from a subset of the multiple motion sensors.

9. The computer-implemented method of any preceding claim, further comprising repeating the fitting to perform readaptation of the adapted biomechanical motion model to account for potential changes in the motion of the object.

10. The computer-implemented method of claim 9, comprising:
changing at least one parameter of the personal biomechanical model template that describes a personal biomechanical characteristic of the object;
performing the fitting again by using the motion measurement data and the personal biomechanical template having the at least one changed parameter, resulting in a newly adapted biomechanical motion model;
determining, on the basis of the newly adapted model, a new value for the at least one parameter; and
outputting the new value of at least one parameter.

11. The computer-implemented method of any preceding claim, wherein the personal biomechanical characteristics comprise at least a mass of the object, body composition of the object, body dimensions of the object, and a fitness metric of the object.

12. The computer-implemented method of any preceding claim, wherein said acquiring the personal biomechanical model template, said fitting the motion measurement data, said determining the at least one parameter, and said outputting are performed by a wrist computer of the object or by a server computer.

13. The computer-implemented method of any preceding claim, wherein said determining the at least one parameter comprises computing, on the basis of motion measurement data acquired from a motion sensor attached to a hand of the object, a contact time of a foot to the ground, and wherein said at least one parameter comprises the contact time.

14. A processing system for modelling biomechanical motion of a human object, comprising:
means for acquiring periodic motion measurement data from a motion sensor located at a determined part of the object while the object is performing periodic motion;
means for acquiring a personal biomechanical model template of the human object, the biomechanical model template defining personal biomechanical characteristics of the object;
means for fitting the motion measurement data with the personal biomechanical model template, the fitting adapting a personal motion style of the object, represented by the motion measurement data, to the personal biomechanical model template, the fitting resulting in an adapted biomechanical motion model describing personalized biomechanical motion of the determined part and at least one other part of the object where no motion sensor is located;
means for determining, on the basis of the adapted biomechanical motion model, at least one parameter describing the biomechanical motion at the other part of the object; and
means for outputting the at least one parameter.

15. A computer program product readable by a computer and comprising program instructions which, when executed by the computer, cause the computer to carry out a computer process implementing all the steps of the method according to any preceding claim 1 to 13.
